# EUROPEAN PATENT SPECIFICATION

(11) **EP 3 917 338 B1**
(45) Date of publication and mention of the grant of the patent: **21.05.2025**
(21) Application number: 20703846.4
(22) Date of filing: 03.02.2020
(51) Int. Cl.: A23L 33/19, A23L 33/00

(54) **NATIVE WHEY PROTEIN FOR IMPROVING INTESTINAL MATURATION**
NATIVES MOLKENPROTEIN FÜR DIE VERBESSERUNG DER GASTROINTESTINALE REIFUNG
PROTÉINE DE LACTOSÉRUM NATIVE POUR AMÉLIORER LA MATURATION GASTRO-INTESTINALE

(30) Priority: 01.02.2019 WO PCT/NL2019/050067
(43) Date of publication of application: 08.12.2021
(73) Proprietor: N.V. Nutricia, 2712 HM Zoetermeer (NL)
(72) Inventor: RENES, Ingrid Brunhilde, 3584CT Utrecht (NL); VERDURMEN, Rudolph Eduardus Maria, 3584CT Utrecht (NL); HOLS, Gerrit, 3584CT Utrecht (NL); ABRAHAMSE, Evan, 3584 CT Utrecht (NL)
(74) Representative: Nederlandsch Octrooibureau
(86) International application number: PCT/NL2020/050059
(87) International publication number: WO 2020/159373

(56) References cited:
- WO-A1-2018/028765
- US-A1- 2014 302 219
- US-A1- 2015 093 490
- US-A1- 2015 125 569
- US-A1- 2015 216 212
- US-A1- 2018 027 864
- YANQI LI ET AL: "Whey Protein Processing Influences Formula-Induced Gut Maturation in Preterm Pigs", THE JOURNAL OF NUTRITION, vol. 143, no. 12, 1 December 2013 (2013-12-01), US, pages 1934 - 1942, XP055622031, ISSN: 0022-3166, DOI: 10.3945/jn.113.182931
- JIN SHI ET AL: "Metabolic effects of a novel microfiltered native whey protein in diet-induced obese mice", JOURNAL OF FUNCTIONAL FOODS, ELSEVIER BV, NL, vol. 4, no. 2, 2 February 2012 (2012-02-02), pages 440 - 449, XP028420323, ISSN: 1756-4646, [retrieved on 20120209], DOI: 10.1016/J.JFF.2012.02.002
- SKOVSTED CILIEBORG M ET AL: "Diet-dependent effects of minimal enteral nutrition on intestinal function and necrotizing enterocolitis in preterm pigs", JPEN - JOURNAL OF PARENTERAL AND ENTERAL NUTRITION, SAGE PUBLICATIONS, INC, US, vol. 35, no. 1, 1 January 2011 (2011-01-01), pages 32 - 42, XP009150540, ISSN: 0148-6071
- SULLIVAN S ET AL: "An Exclusively Human Milk-Based Diet Is Associated with a Lower Rate of Necrotizing Enterocolitis than a Diet of Human Milk and Bovine Milk-Based Products", JOURNAL OF PEDIATRICS, MOSBY-YEAR BOOK, ST. LOUIS, MO, US, vol. 156, no. 4, 1 April 2010 (2010-04-01), pages 562 - 567.e1, XP026975169, ISSN: 0022-3476, [retrieved on 20100318]

## Description

### Field of the invention

The present invention relates to the field of native whey products, in particular infant formulas, for (a) improving intestinal maturation; and/or (b) reducing and/or preventing intestinal permeability.

### Background

Before birth, the foetus receives a constant flow of maternal elemental nutrients via the placenta. After birth, the neonate must adjust to a variable uptake of nutrients from milk, which are available only after specialized digestive processes have occurred in the gastrointestinal tract (GIT). The final maturation of the GIT for enteral nutrient intake occurs shortly before or after term. The digestive system of preterm infants is generally not fully developed, yet such vulnerable infants require additional nutrients for their growth and development. Promoting intestinal maturation and reducing or decreasing intestinal permeability is thought to be beneficial for the development of both term and preterm infants.

Yanqi Li et al, 2013, Journal Nutr, 1934-1942, discloses the effect of several whey protein concentrates on gut-maturation in preterm pigs by investigating the effects based on IL-8 concentrations in the distal small intestine, Gently-treated and minimally-treated whey protein were employed in the tests as alternatives to represent whey protein obtained by different processing methods.

The present invention provides in the need in the art for a native whey protein fraction to be used for improving intestinal maturation, and reducing and/or preventing intestinal permeability, which is fully compatible with the regular enteral feeding regime and suitable for inclusion in infant formulae.

### Summary of the invention

The invention is defined by the claims. The inventors surprisingly found that the native whey protein according to the invention significantly improved intestinal maturation and reduced the occurrence of intestinal permeability. The native whey protein is typically comprised in a nutritional composition, preferably an infant formula such as a preterm formula. The present invention concerns the use of the native whey protein according to the invention for (a) improving intestinal maturation; and/or (b) reducing and/or preventing intestinal permeability.

The whey proteins according to the invention have a nativity of more than 80%, preferably more than 90%, more preferably more than 92%, more than 94%, more than 95% or even more than 98%. Preferably, substantially no non-native whey protein is present in the whey protein. In case the whey protein is comprised in a nutritional composition, this applies to all whey protein in the nutritional composition.

In a preferred embodiment, the whey proteins are intact whey proteins. Intact means that the whey proteins have not been subjected to a hydrolysis step. Thus, substantially no non-intact whey protein is present as whey protein. In case the whey protein is comprised in a nutritional composition, this applies to all whey protein in the nutritional composition.

In one embodiment, the whey protein is comprised in an infant formula and the infant formula is pasteurized or at least the protein fraction thereof is pasteurized. The inventors of the present invention have surprisingly shown that an infant formula with native whey protein, obtained according to the present invention that includes a pasteurization step, can be used for improving intestinal maturation, and reducing and/or preventing intestinal permeability. Alternatively worded, the infant formula is substantially free of alkaline phosphatase activity. In a preferred embodiment, the infant formula is a liquid, ready-to-feed infant formula which is substantially free of alkaline phosphatase activity. In a preferred embodiment, the term substantially free of alkaline phosphatase activity means that, when measured using a liquid, ready-to-feed infant formula, the alkaline phosphatase activity is below 350 mU/L.

In an alternative preferred embodiment, the whey protein is comprised in an infant formula and the infant formula is not pasteurized. The inventors of the present invention have surprisingly shown that an infant formula with native whey protein, obtained according to the present invention without the inclusion of a pasteurization step, can be used for improving intestinal maturation, and reducing and/or preventing intestinal permeability. Alternatively worded the infant formula contains alkaline phosphatase activity. In a preferred embodiment, the infant formula is a liquid, ready-to-feed product and contains alkaline phosphatase activity or is considered alkaline phosphatase positive. The alkaline phosphatase activity of the infant formula in this embodiment is above 350 mU/L.

### Detailed description

The inventors surprisingly found that the native whey protein according to the invention significantly improved intestinal maturation, and reduced the occurrence of intestinal permeability. The native whey protein is preferably comprised in a nutritional composition, more preferably an infant formula. The inventors have developed a process for preparing infant formulae, containing native whey protein.

### Native whey protein

The whey protein according to the invention has an increased nativity compared to whey proteins that are typically comprised in nutritional compositions. The inventors have found that partially or fully replacing the conventional whey protein fraction in such a nutritional composition with the whey protein according to the invention provides a beneficial effect on intestinal maturation and permeability. The present invention does not concern breastfeeding, and the native whey protein in the context of the invention is non-human whey protein. Although any whey protein is suitable in the context of the present invention, the whey protein is preferably bovine whey protein.

The increased nativity of the whey protein according to the invention can be defined in several alternative ways, such as by a nativity of more than 80% and/or by being obtainable by membrane-filtration based technology known per se, preferably by the process according to the invention, in particular step (a).

Thus, the whey protein according to the invention has a nativity of more than 80%, preferably more than 90%, more preferably more than 92%, more than 94%, more than 95% or even more than 98%. In one embodiment, the nativity is in the range of 90 - 100 %, preferably in the range of 94 - 99 %, more preferably in the range of 96 - 99 %. In one embodiment, the nativity is in the range of 90 - 99 %, preferably in the range of 91 - 96 %, more preferably in the range of 92 - 94 %. Being the two most abundant whey proteins, it is especially preferred that α-lactalbumin and β-lactoglobulin have high nativity. The inventors surprisingly found that especially β-lactoglobulin remained largely native in the process according to the present invention. It is thus preferred that α-lactalbuminhas a nativity of at least 70 %, more preferably 75 - 95 %, most preferably 78 - 85 %. Likewise, it is preferred that β-lactoglobulin has a nativity of at least 70 %, more preferably 80 - 100 %, most preferably 85 - 95 %. Without being bound to a theory, it is believed that the nativity of α-lactalbuminand/or β-lactoglobulin, especially β-lactoglobulin, contribute to the beneficial effects on intestinal maturation and permeability.

Nativity is a known parameter in the art and can be determined by any means available to the skilled person. The nativity refers to the percentage of native protein of a particular type based on the total amount of protein of the same type. Here, the nativity of the whey protein refers to the amount of native whey protein based on the total amount of whey protein. In one embodiment, the nativity is determined according to a suitable Kjeldahl-based analysis, such as per the ISO 8968-3 / IDF 20-3:2004, or more preferably according to the procedure in example 3.

The native whey protein according to the invention may contain some other proteins, but preferably not more than 10 wt%, more preferably at most 5 wt%, such as 0.1 - 3 wt%, based on total protein. If other protein is present, this is preferably native casein (micellar casein). Preferably, no denatured caseinate is present. Thus, in one embodiment, the caseinate content of the native whey protein according to the invention is at most 5 wt%, preferably at most 2 wt%, or even at most 0.5 wt%, based on total protein. Most preferably, caseinates are substantially absent from the native whey protein according to the invention.

Additionally or alternatively, the native whey protein according to the invention is defined as being obtainable by membrane-filtration based technology. In a preferred embodiment, the whey does not originate form acid whey or from sweet whey. In other words, the whey has not undergone a step wherein casein is precipitated. It is furthermore preferred that the whey has not undergone a treatment wherein the pH is lowered to a value below 6, preferably not below 5.5. Such lowering of the pH was found to create whey protein aggregates. Such aggregates may negatively affect the beneficial effects of the native whey protein according to the invention on intestinal maturation and permeability. Thus, in one embodiment, the total amount of whey protein aggregates induced by pH-reduction in the native whey protein according to the invention is at most 5 wt%, preferably at most 2 wt%, or even at most 0.5 wt%, based on total whey protein. Most preferably, whey protein aggregates are substantially absent from the native whey protein according to the invention.

A preferred process for preparing the native whey protein, or the nutritional composition comprising the native whey protein is referred herein as the process according to the invention and is further defined below. The inventors have developed a process for making native whey protein, and for making a nutritional composition, in particular an infant formula, comprising the native whey protein.

### Nutritional composition

The native whey protein according to the invention is preferably comprised in a nutritional composition, more preferably in an infant formula, most preferably a preterm formula. The whey protein fraction of the nutritional composition contains at least 80 wt%, preferably at least 90 wt%, of whey proteins being the native whey proteins as defined herein. Most preferably, the nutritional composition is substantially free of whey proteins other than the native whey proteins as defined herein. The nutritional composition according to the invention is preferably for enteral feeding, as that provides the least impact on regular feeding regimes. Nonetheless, the nutritional composition comprising native whey proteins in the form of a tube feed or other feed is also suitable for improving intestinal maturation and reducing and/or preventing intestinal permeability.

In the context of the present invention, "infant formula" refers to milk-based nutritional compositions suitable for feeding infants, which typically are in the form of a reconstitutable powder or a ready-to-feed liquid composition, or refers to infant formula bases, which are suitable for making infant formulae and which comprise all or almost all essential ingredients in the required amounts for infant nutrition. Preferably, the composition is an infant formula, a follow-on formula, a growing-up milk, or a base therefore. Most preferably, the composition is an infant formula. An especially preferred infant formula in the context of the present invention is a preterm infant formula. The infant formula may be a powder, preferably a spray-dried powder, intended to be reconstituted into a liquid infant formula, or a liquid infant formula.

In one embodiment, the nutritional composition comprises native whey protein obtainable by step (a) of the process according to the invention. More preferably, the nutritional composition is obtainable by the process according to the invention. Thus, in one embodiment, the composition according to the present invention comprises a whey protein fraction which is obtainable as a whey protein stream via the process of the present invention as defined below, in particular step (a). In particular, the whey proteins are obtainable by subjecting a defatted, debacterialized milk to microfiltration over a membrane capable of retaining casein and permeating whey proteins to provide a permeate comprising whey protein and fractionating the permeate into a whey protein stream and a lactose stream, wherein debacterization is preferably performed by microfiltration or pasteurization. The whey proteins are present in the thus obtained ultrafiltration retentate. In one embodiment, the whey protein is obtained by the process defined herein. It is well-known to the skilled person how to obtain such an ultrafiltration retentate that contains native whey proteins starting from defatted milk.

In case the nutritional composition is an infant formula, it is typically nutritionally complete for infants, and contains all necessary macronutrients and micronutrients for infant formulas as known in the art. Specifically, the infant formula preferably contains casein, in addition to the native and intact whey protein. The whey protein to casein weight ratio in the infant formula is preferably in the range of 90:10 to 40:60, more preferably in the range of 80:20 to 50:50, even more preferably in the range of 75:25 to 50:50, most preferably in the range of 70:30 to 55:45. In one embodiment, the whey protein to casein weight ratio in the in the infant formula is about 60:40. The exact ratio is typically determined by the type of infant formula that is being produced, and can be adjusted as known in the art.

In one embodiment, the nutritional composition according to the invention shows a negative reaction to an alkaline phosphatase (ALP) activity test. Alternatively, the nutritional composition may exhibit alkaline phosphatase activity. Tests for alkaline phosphate activity are known in the art and are used as standard for defining the activity (or lack of activity) of the enzymes in an infant formula. The law, for example European Regulation 2074/05, and its amendment in EC No 1664/2006, requires the ALP activity to be below 350 mU/L, which is also referred to as ALP negative. The ALP activity can be defined as mU/g (typically for powders, or for liquids based on dry weight) or mU/L (typically for liquids, including reconstituted powders). In case the nutritional composition is a preterm formula, it is preferred that the nutritional composition is ALP negative.

Thus, in one embodiment, the ALP activity of the composition according to the invention, when in liquid form, is below 350 mU/L, or, when in powder form, is typically below 350 mU/L after reconstitution as common in the art of infant formulas. In one embodiment, the composition according to the invention is liquid or reconstituted powder and has an ALP activity in the range of 0 - 350 mU/L, preferably 100 - 350 mU/L, more preferably 200 - 350 mU/L, most preferably 250 - 320 mU/L. Products having such ALP activities may be referred to as denatured and/or deactivated (see e.g. Example 1). In an alternative embodiment, the ALP activity is higher, such as more than 350, or in the range of 350 - 100000 mU/L, preferably 1000 - 50000 mU/L, most preferably 10000 - 30000 mU/L. In one embodiment, the ALP activity is in the range 350 - 450 mU/L. Products having such ALP activities may be referred to as native (see e.g. Example 1) or ALP positive.

In one embodiment, the composition according to the invention has an ALP activity of at most 20 mU/g, preferably at most 5 mU/g, or the composition according to the invention has an ALP activity in the range of 0 - 20 mU/g, preferably 0.1 - 10 mU/g, more preferably 0.2 - 7 mU/g, most preferably 0.5 - 5 mU/g, based on dry weight of the composition. In an alternative embodiment, the composition according to the invention has an ALP activity of at least 25 mU/g, preferably at least 30 mU/g, or the composition according to the invention has an ALP activity in the range of 25 - 150 mU/g, preferably 30 - 50 mU/g, based on dry weight of the composition.

Dairy or milk products with an activity above the indicated ALP threshold value are considered ALP positive in the art and as such qualify as being raw or to comprise raw milk. Even though the ALP positive whey protein of the present invention is associated with milk of raw nature, the present inventors found that it can be used in vulnerable subjects to improve intestinal maturation and intestinal permeability.

In one embodiment, the ALP activity is determined by ISO standard 11816-1. Alternatively, the ALP activity is determined by the following procedure. A solution of the whey protein, typically as a 10 wt% protein solution, is mixed with an equal amount of 1-butanol, and the mixture is then centrifuged between 2500-3500 g for 30 min. The aqueous phase is collected from beneath the fat layer and diluted between 1/5 - 1/200. These sample solutions were added in the wells of an enzyme-linked immunosorbent assay (ELISA) plate, coated with a monoclonal antibody specific to the alkaline phosphatase found in cow's milk, together with control and standard solutions. The plates are incubated for 1 h at 18 - 25 °C, after which the solutions are removed from the wells and substrate solution is added to each well. The plates are incubate for 2 h at 35 - 38 °C. After stopping the incubation, the plate is imaged at a wavelength of 405 nm, and the ALP activity is determined by comparison of the optical density of the sample with that of the standard. In an especially preferred embodiment, the nutritional composition according to the invention comprises intact whey protein, wherein at least 90 % of the whey protein is native and the ALP activity is in the range of 100 - 350 mU/L, as determined by ISO standard 11816-1. Preferably, this nutritional composition is obtainable by the process defined herein in case a pasteurization step is included to provide a debacterialized milk. In an alternative preferred embodiment, the nutritional composition according to the invention comprises intact whey protein, wherein at least 90 % of the whey protein is native and the ALP activity is higher than 350 mU/L or in the range of 350 - 100000 mU/L, as determined by ISO standard 11816-1. Preferably, this nutritional composition is obtainable by the process defined herein in case a microfiltration step is included using a membrane capable of retaining bacteria and permeating milk proteins to provide a debacterialized milk.

### The process for making native whey protein or the nutritional composition

In one embodiment, the native whey protein is obtainable by membrane-filtration based technology in order to retain the nativity of the whey proteins. Preferably, the process for obtaining the native whey proteins comprises:
(a) processing defatted milk into a casein stream, a whey protein stream and a lactose stream, by:
   (i) subjecting the defatted milk to microfiltration over a membrane capable of retaining bacteria and permeating milk proteins or to a pasteurization step, to provide a debacterialized milk;
   (ii) subjecting the permeate originating from step (i) to microfiltration over a membrane capable of retaining casein and permeating whey proteins, to provide a casein stream as retentate and a permeate comprising whey protein;
   (iii) fractionating the permeate originating from step (ii) into a whey protein stream and a lactose stream.

In one embodiment, the nutritional composition is obtainable by the process comprising:
(a) processing defatted milk into a casein stream, a whey protein stream and a lactose stream, by:
   (i) subjecting the defatted milk to microfiltration over a membrane capable of retaining bacteria and permeating milk proteins or to a pasteurization step, to provide a debacterialized milk;
   (ii) subjecting the permeate originating from step (i) to microfiltration over a membrane capable of retaining casein and permeating whey proteins, to provide a casein stream as retentate and a permeate comprising whey protein;
   (iii) fractionating the permeate originating from step (ii) into a whey protein stream and a lactose stream;
(b) combining at least part of the casein stream, at least part of the whey protein stream originating from step (a) and a lactose source to obtain a recombined stream;
(c) optionally pasteurization of the recombined stream from step (b),
(d) using the recombined stream originating from step (b) or (c) in the manufacture of the nutritional composition.

In the process according to the invention defatted milk is treated to produce an nutritional composition. In the context of the present invention, whenever a certain stream or composition is mentioned to "originate from" a certain process step, such as from the recombined stream originating from step (b), said stream or composition can be the composition which is directly obtained by said process step. In addition, if such a directly obtained stream or composition undergoes one or more additional processing steps, such as partial evaporation and/or supplementation of additional water or other components, the stream or composition is also regarded to originate from that specific process step. Thus, if the recombined stream of step (b) would be partially evaporated prior to it is entered in the pasteurization step (c), the incoming stream of step (c) is still regarded to be the recombined stream originating from step (b). In the context of the present invention, the term "stream" refers to a liquid composition, although the presence of some solid material is not excluded, e.g. as in a suspension, as long as the composition can be handled by conventional dairy plants.

The present process uses milk as starting material in step (a). Defatted milk, preferably defatted cow's milk, is subjected to step (a). In the context of the invention, "defatted milk" refers to milk having a reduced fat content compared to whole milk. Typically, the fat content of the defatted milk is in the range of 0 - 2 wt%, preferably 0 - 1 wt%, more preferably 0 - 0.2 wt%, most preferably 0 - 0.05 wt%, based on total weight of the defatted milk. In one embodiment, the defatted milk is skim milk. The present process employs milk, which refers to non-human milk, preferably cow's milk. Most preferably, cow's skim milk is used. In one embodiment, the process comprises a step of defatting milk to obtain the defatted milk, which is subsequently subjected to step (a). Herein, non-defatted milk, or just milk or whole milk, is subjected to the defatting step. The defatting step affords the defatted milk. Preferably, the defatted milk is the sole protein source for the nutritional composition.

### Step (a)

In step (a), the defatted milk is processed or fractioned into a casein stream, a whey protein stream and a lactose stream. Herein, the casein stream is a liquid composition comprising casein, which is enriched in casein compared to the casein content in the incoming defatted milk, the whey protein stream is a liquid composition comprising whey protein, which is enriched in whey protein compared to the whey protein content in the incoming defatted milk and the lactose stream is a liquid composition comprising lactose, which is enriched in lactose compared to the lactose content in the incoming defatted milk. In the context of the present invention, "enriched" is defined that the content of the enriched component, based on dry weight, is increased in one stream compared to another stream. Thus, the casein stream is enriched in casein, i.e. has a higher casein content, based on dry matter, compared to the incoming defatted milk.

The fractionation of step (a) is accomplished by membrane filtration techniques and involves a combination of microfiltration and ultrafiltration. The casein stream originates from the microfiltration as retentate, the whey protein stream originates from the ultrafiltration as retentate and the lactose stream originates from the ultrafiltration as permeate. Suitable membrane filtration processes are known in the art, e.g. as disclosed in WO 2013/068653, WO 2013/137714 and WO 2015/041529. More specifically, step (a) includes:
(i) subjecting the defatted milk to microfiltration over a membrane capable of retaining bacteria and permeating milk proteins or to a pasteurization step, to provide a debacterialized milk;
(ii) subjecting the permeate originating from step (i) to microfiltration over a membrane capable of retaining casein and permeating whey proteins, to provide a casein stream as retentate and a permeate comprising whey protein; and
(iii) fractionating the permeate originating from step (ii) into a whey protein stream and a lactose stream.

The incoming defatted milk is subjected to debacterization (bacterial removal) in step (i). Debacterization may be performed by filtration or by pasteurization. In one embodiment, debacterization is performed by bacterial filtration (e.g. microfiltration (MF)). Such filtration processes to reduce the bacterial load of milk are known in the art. The microfiltration of step (i) may be performed by microfiltration over a membrane capable of retaining bacteria and permeating milk proteins, to provide a debacterialized milk as permeate. Preferably, the microfiltration of step (i) comprises ceramic microfiltration. The MF membrane preferably has a pore size of between 1.8 and 0.6 µm, preferably between 1.4 and 0.8 µm. The MF process of step (i) is preferably executed at a temperature of between 4 and 20 °C, more preferably between 8 and 15 °C, most preferably at a temperature of about 10 °C.

Alternatively, step (i) is performed by pasteurization. Pasteurization of defatted milk in order to reduce the bacterial load of the milk is well-known in the art. Pasteurization and preferred embodiments thereof are described in more detail below in the context of step (c), which equally applies here.

In the microfiltration step (ii), the debacterialized milk originating from step (i) is fractioned into two distinct streams, each enriched in a particular protein type; a casein enriched MF retentate (MFR) and a whey protein enriched MF permeate (MFP) are produced. The MF step (ii) is performed over a membrane that enables fractionation of casein and whey proteins. Such a membrane typically has a porosity of between 0.05 and 0.5 µm, more preferably between 0.08 - 0.35 µm. Alternatively, the membrane used in step (ii) may have a molecular weight cut-off in the range of 250 - 1500 kDa, preferably in the range of 500 - 1000 kDa. Preferably, a ceramic membrane or a spiral wound (organic) membrane is used. Microfiltration of step (ii) is preferably performed with a volume concentration factor (VCF) in the range of 1.5 - 10, preferably 2 - 5, which has been found to provide the most optimal results in terms of the composition of the MF retentate, especially in terms in terms of casein content.

In the context of the invention, the term "volume concentration factor" or "VCF" is the factor at which a liquid composition is concentrated upon filtration, i.e. the total volume of the incoming stream prior to filtration divided by the total volume of the retentate after filtration, irrespective of the total solid content. Thus, when 5 L of a liquid composition is fractionated over an ultrafiltration membrane into a permeate of 4 L and a retentate of 1 L, this UF process operates with a VCF of 5/1 = 5.

According to a preferred embodiment, microfiltration of step (ii) is enhanced with diafiltration (DF). Diafiltration may be accomplished by diluting the retentate of the MF at least once with an amount of water, or by diluting the incoming debacterialized milk with an amount of water and subjecting the diluted milk to MF. The DF water may be added to the incoming debacterialized milk or MFR at once, or the total amount of DF water may be added in several fractions. After each addition of DF water to the incoming skim milk or MFR, the diluted liquid composition is subjected to MF.

Fractionation of a composition comprising whey protein and lactose into a composition enriched in whey protein and a composition enriched in lactose is known in the art. Step (iii) is preferably performed by ultrafiltration (UF). During ultrafiltration, most of the liquid and small solutes end up in the UF permeate (UFP), while the UF retentate (UFR) comprises substantially all whey protein, in a smaller volume. Small molecules which permeate through the UF membrane are for example lactose, monovalent and polyvalent ions. The ultrafiltration of step (iii) can be carried out with any UF membrane known in the art, including ceramic membranes, tubular and organic spiral wound membranes. Preferably the UF membrane is an organic spiral wound membrane. The UF membrane has a molecular weight cut-off of that enables proteins, preferably whey proteins, to remain in the retentate, and allow small solutes, for example lactose, to permeate through the membrane. The UF step (iii) preferably is carried out with a membrane having a molecular weight cut-off of at most 25 kDa, more preferably at most 10 kDa, and preferably of at least 2.5 kDa, more preferably at least 5 kDa. The UF step (iii) is preferably carried out with a volume concentration factor (VCF) in the range of 20 - 200, preferably 50 - 150, which has been found to provide the most optimal results in terms of the composition of the UF retentate.

Step (a) may further comprise one or more concentration steps, such as concentration of the MFR originating form step (ii) and/or the UFR originating form step (iii). Concentration is preferably performed by reverse osmosis (RO), nanofiltration (NF) and/or evaporation. NF is most preferred, as NF concentrates the stream and at the same time lowers the monovalent ion content, which are able to permeate the NF membrane. Such lowering of the monovalent ion content is especially desirable in the production of infant formulas.

The protein fraction of the casein stream originating from step (a) typically comprises very little whey protein, preferably less than 15 wt%, more preferably less than 10 wt%, based on the weight of the protein fraction of the casein stream, and is high in casein. Preferably the protein fraction comprises at least 85 wt% casein, more preferably at least 90 wt% casein. The content of total solids in the casein stream typically ranges from 5 to 30 wt%, preferably from 7 to 30 wt%, most preferably from 17 to 24 wt%, based on total weight of the casein stream. The casein stream may also be referred to as a casein concentrate, casein isolate, micellar casein concentrate or micellar casein isolate (MCI).

The whey protein stream is typically a liquid composition having a total solid content of 5 - 35 wt%, preferably of 10 - 30 wt%, most preferably of 20 - 30 wt%, and typically comprises 25 - 90 wt%, preferably 60 - 85 wt% whey proteins based on total dry weight. The whey protein stream may also be referred to as an aqueous composition comprising whey proteins. Although the whey protein stream is enriched in whey protein compared to the incoming defatted milk, it may still contain substantial amounts of casein, depending on the exact conditions at which the fractionation between casein and whey protein by ultrafiltration, is performed. In one embodiment, the whey protein stream comprises at most 40 wt%, preferably 5 - 20 wt% casein, based on total weight of the protein. Such variations in the fractionation conditions and the accompanying changes in the whey protein stream are known in the art. Depending on the amount of casein present in the whey protein stream, the amount of casein used in combining step (b) can be adapted such that the nutritional composition has a whey protein: casein ratio that falls within the preferred ratio of 90:10 to 40:60.

The lactose stream is typically a liquid composition having a total solid content of 3 - 30 wt%, preferably of 5 - 22 wt%. The lactose content in the lactose stream originating from step (a) is typically at least 75 wt%, preferably at least 90 wt% or even at least 95 wt%, based on total dry weight.

### Demineralization

The process according to the invention preferably comprises a demineralization step, wherein the lactose source, or one or more components thereof, is/are demineralized prior to being subjected to step (b). Demineralization is thus typically performed on at least part of the lactose stream originating from step (a) prior to being subjected to step (b). Demineralization is particularly preferred for the manufacture of infant formulas, for which it is typically required to lower the mineral content as compared to the incoming milk. Thus, in one embodiment, at least part of the lactose stream originating from step (a), preferably the UFP originating from step (iii), is subjected to demineralization prior to being used as (part of) the lactose source in step (b).

Demineralization of the lactose source may be performed by any technique known in the art, such as electrodialysis, ion exchange, salt precipitation, lactose crystallization, membrane filtration techniques such as nanofiltration, optionally enhanced with diafiltration, or combinations thereof. In a preferred embodiment, demineralization comprises at least one of salt precipitation, electrodialysis, lactose crystallization and ion exchange, optionally in combination with nanofiltration, more preferably demineralization comprises nanofiltration in combination with at least one of salt precipitation, electrodialysis, lactose crystallization and ion exchange. In preferred embodiment, demineralization comprises at least electrodialysis and/or salt precipitation. In one preferred embodiment, demineralization comprises at least nanofiltration in combination with electrodialysis and/or salt precipitation. The inventors found that when only nanofiltration is used for demineralization, especially for demineralization of an ultrafiltration permeate as lactose source in the preparation of infant formulas, the content of divalent ions, such as calcium and phosphate, is typically insufficiently reduced to obtain a final infant formula within legal requirement.

Demineralization is preferably performed such that at least 20 wt%, or preferably 50 wt%, more preferably at least 70 wt% or at least 80 wt%, most preferably at least 90 wt% of the polyvalent ions and/or such that at least 20 wt% of the monovalent ions are removed, more preferably at least 35 wt% or at least 50 wt%, most preferably at least 60 wt% of the monovalent ions, present in the lactose stream, e.g.t the UFP originating from step (iii), are removed.

### Step (b)

In step (b), at least part of the casein stream, at least part of the whey protein stream originating from step (a) and a lactose source are combined to obtain a recombined stream. This recombined stream is used to manufacture the nutritional composition in step (d), optionally after a pasteurization step (c). The combining of step (b) affords a composition having a protein fraction comprising both casein and whey protein in a certain weight ratio. The combining of step (b) may involve additional components. The combining is preferably done such that the whey protein to casein weight ratio in the recombined stream is in the range of 90:10 to 40:60, more preferably in the range of 80:20 to 50:50, even more preferably in the range of 75:25 to 50:50, most preferably in the range of 70:30 to 55:45. In one embodiment, the whey protein to casein weight ratio in the recombined stream is about 60:40. The exact ratio is typically determined by the type of nutritional composition, in particular the type of infant formula that is being produced, and can be adjusted as known in the art. In addition, much attention in the art is given to the amino acid profile of infant formulas. The process according to the invention provides optimal flexibility in targeting a specific desired amino acid profile, e.g. by adjusting the ratio in which the whey protein and casein streams are combined or in varying the specific process conditions of the microfiltration of step (a). As such, optimal amino acid profiles resembling those found in human milk are obtainable with the process according to the invention.

In one embodiment, 10 - 50 wt%, preferably 12 - 25 wt%, based on total weight of the casein, of the casein stream originating from step (a) is subjected to step (b). Most preferably, about 16 wt%, based on total weight of the casein, of the casein stream originating from step (a) is subjected to step (b). The amount of the casein stream originating from step (a) that is subjected to step (b) is advantageously governed by the desired whey protein to casein weight ratio in the recombined stream. Preferably, all of the whey protein stream originating from step (a) is subjected to the combining of step (b). In one embodiment, 0 - 50 wt%, preferably 5 - 25 wt%, based on total weight of the lactose, of the lactose stream originating from step (a) is subjected to step (b) as (part of) the lactose source. The amount of the lactose stream originating from step (a) that is subjected to step (b) as (part of) the lactose source is advantageously governed by the amount of lactose required for step (d). In case the amount of lactose in the lactose stream originating from step (a) that is subjected to step (b) would be insufficient for infant formula manufacture, additional lactose can be used. In one embodiment, part of the casein stream is combined with all of the whey protein stream and part of the lactose stream. In one embodiment, part of the casein stream is combined with all of the whey protein stream and all of the lactose stream. In one embodiment, part of the casein stream is combined with all of the whey protein stream and nothing of the lactose stream. In one embodiment, part of the MFR originating from step (ii) is combined with at least part of the UFR originating from step (iii) and at least part of the UFP originating from step (iii).

In step (b), three or more streams are recombined into one stream. This recombining may occur at once (streams are combined simultaneously) or step-wise (streams are combined consecutively). Combining can be performed as wet mixing or as dry mixing or even as a combination of both. Preferably, the combining occurs as wet mixing, wherein liquid compositions are mixed in the appropriate amounts.

### Step (c)

The process according to the invention may contain a pasteurization step, although omitting the pasteurization step also affords suitable products. If a pasteurization step is performed, it may be performed as step (i) or as step (c). In a preferred embodiment, a pasteurization step is performed, since this is a requirement for infant formulas in many jurisdictions from a food safety perspective. In a preferred embodiment, the process of the invention contains only a single pasteurization step to ensure the obtained product is sufficiently heat-treated with regards to prevention of microbial or bacterial contaminations but on the other hand ensures preservation of protein nativity. Thus, in a preferred embodiment, step (i) is a pasteurization step and step (c) is not performed, or step (i) is a filtration step and step (c) is performed. Although the incoming defatted milk may be pasteurized in step (i), it is preferred that if a pasteurization step is included, the recombined stream originating from step (b) is subjected to a pasteurization step (c) prior to being subjected to step (d). Alternatively, no pasteurization step is performed and step (i) is performed by filtration and step (c) in omitted. By virtue of filtration step (i), the thus obtained products are sufficiently debacterized to be suitable in the context of the present invention. Most preferably, step (c) is performed in case debacterialization in step (i) is achieved by microfiltration.

Pasteurization is known in the art and may e.g. involve HTST, ESL or UHT. The pasteurization step as meant herein has the purpose of reducing the microbial load to such an extent that the resulting nutritional composition is free from microorganisms and safe for consumption, even by infants. In particular, it is safe with regards to *Bacillus cereus* and *Enterobacter sakazakii,* for instance, such as laid down in European Regulation No 2073/2005 dated 2007, corrigendum No. 1441/2007. Preferably, pasteurization involves heating at 72 - 74°C for 15 to 30 seconds, or, alternatively, a heat-treatment equivalent thereto, meaning that the same heat load is applied, as is known to the skilled person. Preferably, the equivalent heat-treatment results in the same reduction in bacterial load and preserves the protein nativity to the same extent as a pasteurization step at 72 - 74°C for 15 to 30 seconds, resulting in whey proteins having a nativity of more than 90%, preferably more than 95 or even more than 98%.

### Step (d)

In step (d), the recombined stream originating from step (b) is used to manufacture the nutritional composition. Such manufacturing is known in the art and typically involves one or more of drying, concentrating, supplementing with vitamins, minerals, lipids and/or dietary fibres, heat treatment, homogenisation, packaging. In a preferred embodiment, step (d) does not involve heat treatment, and involves one or more of drying, concentrating, supplementing with vitamins, minerals, lipids and/or dietary fibres and packaging. Preferably, step (d) involves at least a drying step, most preferably it involves all of the above mentioned steps. In a preferred embodiment, a drying step is performed directly after step (b) or (c), most preferably directly after step (c).

Although one or more of the separate streams may be dried prior to being combined in step (b), it is preferred that the recombined stream originating from step (b) is dried, preferably spray-dried. As such, only one drying step is needed in the manufacture of the nutritional composition. In a preferred embodiment, the process according to the invention comprises only a single drying step, wherein in step (d) the recombined stream is dried, preferably by spray-drying. Due to the inherently limited heat-load as a consequence of low water activity of droplets produced during spray-drying, protein nativity remains substantially the same and is not significantly impacted during spray-drying. This allows that the content of native protein in the final nutritional composition is as high as possible and substantially the same as prior to spray-drying. To retain the native protein content in the final product, the spray-drying step is preferably executed with an inlet temperature of less than 250 °C, preferably less than 220 °C, more preferably less than 200 °C. Alternatively worded, the spray-drying step is executed such that the wet bulb temperature is kept below 80°C, preferably below 70° C or even below 50 °C. Using such spray-drying conditions, nativity of the proteins that are spray-dried will not be impacted anymore due to the low water activity of the powder particles in the spray-drier. In one embodiment, the recombined stream is concentrated, preferably prior to being dried. Such concentration may be accomplished by any means known in the art, such as by reverse osmosis (RO), nanofiltration (NF) and/or evaporation.

Depending on the desired type of nutritional composition, supplementation of certain components, such as vitamins, minerals, lipids and/or dietary fibres, may be desired. Such supplementation can be performed either prior to, during or after combining step (b) and/or optionally prior to or after a drying step. The skilled person is aware of the requirements of particular types of nutritional compositions, especially infant formulas, e.g. from EU directive 91/321/EEC or EU directive 2006/141/EC or US Food and Drug Administration 21 CFR Ch 1 part 107, and is able to adjust the composition of the recombined stream in order to meet those requirements.

As will be appreciated by the skilled person, process steps that lead to denaturation of the whey protein should be avoided as much as possible. For example, the nutritional composition may be a spray-dried powder, in which case it is preferred that that the spray-drying step is executed with an inlet temperature of less than 250 °C, preferably less than 220 °C, more preferably less than 200 °C. It is preferred that the whey proteins have undergone a pasteurization step preferably a single pasteurization step.

### Application

The inventors surprisingly found that the native whey protein according to the invention has beneficial effects on the gut, in particular regarding gut maturation and intestinal permeation. The inventors surprisingly found a significantly reduced lactulose/mannitol ratio in the urine (see Example 6), which is a clinical marker for reduced gut permeability. The lactulose to mannitol ratio measures intestinal permeability based on lactulose being too large for intracellular diffusion yet relying on paracellular diffusion for intestinal absorption whereas mannitol absorption is less dependent on intestinal integrity. Thus, the native whey protein according to the invention is capable of (a) improving intestinal maturation; and (b) reducing and/or preventing intestinal permeability. In the context of the present invention, the intestine includes at least the colon, most preferably is the colon.

The improvement of intestinal maturation and/or reduction in and/or prevention of intestinal permeability, caused by the native whey proteins according to the invention, occurs with respect to the whey proteins that are typically present in nutritional compositions such as infant formula, which are not native or native to a much lesser extent. Thus, in one embodiment, the improvement of intestinal maturation and/or reduction in and/or prevention of intestinal permeability occurs with respect to the occurrence thereof in a subject fed the same whey protein which is made non-native, preferably by sufficient heating to reach a nativity of less than 20%.

In the context of the present invention, intestinal permeability refers to the medical condition, wherein the intestinal wall is permeable for components present in the intestine which should normally not pass the intestinal wall (e.g. food particle, microorganisms, toxins). Normal functioning of the intestinal wall, including permeation of nutrients, is not affected by the use of native whey protein according to the invention. Thus, in the context of the present invention "intestinal permeability" can also be referred to as "impaired intestinal permeability". Intestinal permeability has been found to contribute to the development of several diseases, such as necrotizing enterocolitis, Crohn's disease, celiac disease, diabetes, intestinal inflammation, irritable bowel syndrome, obesity, fatty liver disease and food intolerance. The present invention thus provides a reduction in the occurrence of diseases associated with intestinal permeability or a reduced predisposition for diseases associated with intestinal permeability. This is particularly relevant for subjects having an immature intestine, such as preterm infants. It is particularly preferred that the native whey protein according to the invention provides a reduced predisposition for food intolerance and/or intestinal inflammation, such as necrotizing enterocolitis.

In the context of the present invention, the native whey protein, typically as comprised in a nutritional composition as defined above, is administered to a subject at risk thereof and/or in need thereof. The subject may thus be at risk, or even increased risk, of developing diseases associated with intestinal permeability, such as those defined above. The subject is typically an infant, preferably a human infant. Preferably, the infant is a preterm infant. Preferably, the infant is 0 - 36 months of age, more preferably 0 - 12 months of age, even more preferably 0 - 6 months of age, even more preferably 0 - 4 months of age, most preferably 0 - 2 months of age. In case of preterm infants, this age refers to the age of the actual birth date.

In a preferred embodiment, the subject is in need of improving intestinal maturation or reducing and/or preventing intestinal permeability. In one embodiment, the subject has an immature intestine, in particular an immature intestine in need of maturation. In one embodiment, the subject is at risk of developing intestinal permeability. In a preferred embodiment, the subject is a preterm infant. Preterm infant, or premature infant, refers to an infant that was born before the 37^{th} week of gestation. Such infants are characterized by having immature organs and tissues due to the premature birth. Among others, the intestines are characterized as being immature and in need of maturation.

The infant formula according to the invention is typically suitable as complete nutritional product for infants, like regular infant formula. Administration of the infant formula according to the invention occurs as (part of) the regular feeding regime of the infant. In one embodiment, the use according to the invention is further for providing nutrition to the infant.

The invention thus concerns native whey protein for use in (a) improving intestinal maturation; and/or (b) reducing and/or preventing intestinal permeability.

### Description of the Figures

The figures depict the results of Example 6.
Figure 1 depicts the Lac/Man ratio (L/M) of the near term group, wherein P = PAST-WPC diet according to the invention and D = DENAT-WPC control diet.
Figure 2A depicts the crypt depths in µm of the preterm group (PT) and the near term group (NT). Figure 2B depicts the Ki67 positive proliferating cells per crypt, observed for the preterm group (PT) and the near term group (NT). Figure 2C depicts the number of animals with a histological positive alkaline activity, observed for the preterm group (PT) and the near term group (NT), wherein negative is white and positive is shaded. Herein, P = PAST-WPC diet according to the invention and D = DENAT-WPC control diet.
Figure 3A depicts the ALP activity (in units) for the preterm group (PT) and the near term group (NT). Figure 3B depicts the same ALP activity, but after specific blockage of iALP (preincubation with L-PHE). Herein, P = PAST-WPC diet according to the invention and D = DENAT-WPC control diet.
Figure 4 depicts the enteroendocrine cell count as 5HT⁺ cells per crypt (y-axis) for the near term group. Herein, P = PAST-WPC diet according to the invention and D = DENAT-WPC control diet.

### Examples

The following examples illustrate the invention.

### Example 1: WPC70 preparation

Three WPC70 products, (i) native WPC70, (ii) deactivated WPC70, and (iii) denatured WPC70, were prepared according to the following process. Milk and subsequent fractions were stored at 4 °C throughout production. Whole raw milk (purchased from Dairygold) was skimmed using typical GEA Westfalia Separator @ 55 °C and cooled to 4 °C. Skim milk was subjected to microfiltration to separate casein from both whey and lactose. Microfiltration membrane used was a 0.08 µM Synder membrane FR (PVDF 800kDa) spiral wound membrane. The microfiltration retentate (MFR) was kept as the casein fraction and the microfiltration permeate (MFP) contained whey, lactose and ash. The operating temperature was 10 °C and volume concentration factor (VCF) was 3. This VCF factor was optimal to obtain the required final concentration of casein protein in the MFR. The MFP was then subjected to ultrafiltration to separate whey protein from lactose at operating temperature of 10 °C with VCF of 90. This VCF factor gave an optimal final concentration of whey protein in ultrafiltration retentate (UFR). A native WPC70 was produced. The ultrafiltration membrane used was a 10kDa Synder membrane ST (PES 10kDa) spiral wound membrane. Diafiltration medium was added to improve separation efficiency of membranes (200% of original starting skim milk volume). Concentrated liquid WPC70 (DM 11%) was stored at 4 °C until further handling. The WPC70 was heated to 30 °C and spray dried at 11% DM. The spray-dryer used was a single stage pilot scale dryer operated with an inlet temperature of 185 °C and outlet temperature of 90 °C. This sample is referred to as the native WPC70 and represents a highly native, alkaline phosphatase positive sample.

Deactivated WPC70 was prepared to represent a highly native, pasteurized protein sample which can be included in an infant formula. It was prepared by re-hydrating the native WPC70 in 40 °C RO water using a high speed mixer for 30 min, resulting in a total solids content of 10% and a protein content of about 7%. This solution was heat-treated at 73 °C / 30 s using a Microthermics tubular heat exchanger (MicroThermics, North Carolina, USA). The heat-treated WPC was then freeze-dried resulting in a WPC70 powder with inactivated bioactive components, indicated by the inactivation of alkaline phosphatase, and whey protein nativity of > 95 %.

Denatured WPC70 was prepared by re-hydrating the native WPC70 in 40 °C RO water using a high speed mixer for 30 min, resulting in a total solids content of 10% and a protein content of about 7 %. This solution was heat treated at 100 °C / 60 s using a Microthermics tubular heat exchanger (MicroThermics, North Carolina, USA). The heat-treated WPC was then freeze-dried resulting in a WPC70 powder with whey protein nativity of < 30 %.

The composition of the three WPC70 products, as a 7 % protein solution (see Example 3), is given in the table below (in wt% based on dry weight):

| | **Total Protein** | **NPN** | **NCN** | **True protein** | **Casein** | **Whey** | **Nativity** |
|---|---|---|---|---|---|---|---|
| **Native WPC70** | 7.13 | 0.15 | 5.33 | 6.97 | 1.65 | 5.30 | 100 % |
| **Deactivated WPC70** | 7.11 | 0.15 | 5.23 | 6.96 | 1.73 | 5.08 | 95.73 % |
| **Denatured WPC70** | 6.70 | 0.16 | 1.16 | 6.54 | 5.38 | 1.14 | 21.43 % |

### Example 2: IMF preparation

Three IMF products, (i) Native IMF, (ii) Deactivated IMF, and (iii) denatured IMF, were prepared according to the following process. The wet phase of the infant milk formulation was prepared by first dissolving lactose powder in 90 °C RO water with agitation provided by a high speed silverson mixer (Silverson^{®}, Chesham Bucks, U.K). The solution was cooled to 45 °C, micellular casein concentrate (MCC, MFR obtained in Example 1) and native whey protein concentrate (native WPC70 obtained in Example 1) were added to the solution, allowing for a final casein: whey ratio of 40:60 (similar to the ratios observed in mothers milk), and re-hydrated under high speed mixing for 20 min. Galacto-oligossaccharide (GOS) syrup was added to the mix once the casein and whey protein powders had sufficiently hydrated and mixed for 15 min. Micronutrient components were added to the macronutrients as per a pre-determined recipe. All ingredients were added and agitated at high speeds for 20 min.

For native IMF, the wet phase was directly combined with a pre-prepared oil blend and homogenized via the addition of soy lecithin powder and high speed agitation for 20 min. This completed IMF (50-55 % TS) was subjected directly to a multi-stage Anhydro spray-dryer (water evaporation capacity (WEC) 30 kg/hr) operated with an inlet temperature of 185 °C and an outlet temperature of 90 °C, resulting in a powdered native IMF with < 4 % moisture.

Deactivated IMF was manufactured by pasteurizing the wet phase using a Microthermics tubular heat exchanger (MicroThermics, North Carolina, USA) at 73 °C / 30 s. The pasteurized wet phase was combined with a pre-prepared oil blend and homogenized via the addition of soy lecithin powder and high speed agitation for 20 min. This pasteurized compound was dried using a Single stage pilot dryer (WEC 10 kg/hr) which produced a deactivated IMF with a whey protein nativity of > 95 % and inactivated bioactive components as indicated by the inactivation of the enzyme alkaline phosphatase.

Denatured IMF was prepared by rehydration of native IMF powder to a protein content of about 10 %. This compound was mixed at high speed for 30 min to ensure full dissolution. The compound was then heat-treated using a Microthermics tubular heat exchanger (MicroThermics, North Carolina, USA) at 100 °C / 60 s. The heat-treated compound was collected and freeze-dried to produce a denatured IMF with a whey protein nativity of < 40 %.

The composition of the three IMF products, as a 10 % protein solution (see Example 3), is given in the table below (in wt% based on dry weight):

| | **Total Protein** | **NPN** | **NCN** | **True protein** | **Casein** | **Whey** | **Nativity** |
|---|---|---|---|---|---|---|---|
| **Native IMF** | 10.19 | 0.41 | 6.44 | 9.78 | 3.34 | 6.38 | 100% |
| **Deactivated IMF** | 10.05 | 0.40 | 6.25 | 9.65 | 3.40 | 6.19 | 97.03% |
| **Denatured IMF** | 9.98 | 0.40 | 2.43 | 9.59 | 7.15 | 2.37 | 37.16% |

### Example 3: Nativity calculation

The total nitrogen (TN), non-protein nitrogen (NPN) and non-casein nitrogen (NCN) were determined via kjeldahl analysis, as per the ISO 8968-3 / IDF 20-3:2004 standard (MilkDetermination of nitrogen content -- Part 3: Block-digestion method (Semi-micro rapid routine method), 2004), using an automatic Kjeltec 8400 unit (FOSS, Warrington, U.K). The nativities of the whey proteins in Examples 1 and 2 were calculated as follows:
(a) Casein fraction = (TP - NPN) - NCN
(b) Whey fraction = NCN - NPN
(c) Nativity = measured whey fraction (b) / theoretical whey faction * 100 % The theoretical whey fraction is based on the casein / whey protein ratio of the product, from the recipe of the product.

### Example 4: Determination of alkaline phosphatase activity

Alkaline phosphatase (ALP) activity in native and heat treated WPC and IMF products was determined using an immunocapture assay using specialized ALP assay kits (IDBiotech, Rue Marie Curie, Issoire, France). The kit contained an enzyme-linked immunosorbent assay (ELISA) plate coated with a monoclonal antibody specific to the alkaline phosphatase found in cow's milk. 1-butanol is the solvent used for enzyme extraction. Enzyme activity is expressed as equivalent-milliunit per litre (Eq.mU/I).

Sample preparation: 3 ml of WPC (10 % protein) or IMF (10 % protein) was mixed with 3 ml of 1-butanol, capped and mixed using a vortex for 30-40 s. Samples were then centrifuged between 2500-3500 g for 30 min. The aqueous phase was collected from beneath the fat layer and diluted between 1/5 - 1/200 (recommended) using the dilution buffer provided.

A standard solution was prepared as per the instructions within the assay kit, resulting in a working solution of 15,000 Eq.mU/I which in turn was diluted using the provided dilution buffer to create standards varying in concentration from 5,000-100 Eq.mU/I:
- STD1 5000 Eq.mU/I: 125 µl (15,000 Eq.mU/I) + 250 µl dilution buffer;
- STD2 3000 Eq.mU/I: 75 µl (15,000 Eq.mU/I) + 300 µl dilution buffer;
- STD3 1000 Eq.mU/I: 25 µl (15,000 Eq.mU/I) + 350 µl dilution buffer;
- STD4 500 Eq.mU/I: 50 µl (STD1) + 450 µl dilution buffer;
- STD5 300 Eq.mU/I: 50 µl (STD2) + 450 µl dilution buffer;
- STD6 100 Eq.mU/I: 50 µl (STD3) + 450 µl dilution buffer.

To run the assay: each well of the ELISA strips was washed with 300 µl of wash buffer, which was removed by inverting the plate. This is repeated 4 times. 100 µl of standard, control and sample solutions were added to the corresponding wells, the plate was covered and was shaken gently for 1 min and incubated for 1 hour at 18 - 25 °C. After incubation, the standard, control and sample solutions were removed from the wells (by inversion of the plate) and the wash step as described above was performed. 100 µl of substrate solution was added to each well. The plate was covered, shaken gently for 1 min and incubate for 2 hrs at 35- 38 °C. A yellow colour develops. 50 µl of stop solution (provided in the kit) was added to all wells after incubation. The plastic cover was removed and the plate was read at 405 nm using a microplate reader. A calibration curve was obtained by plotting the optical density reading for the standard samples and this curve was used to determine the alkaline phosphatase activity in the WPC and IMF products.

The results are given in the table below:

| | **ALP activity** |
|---|---|
| **Native WPC70** | 195 mU/g |
| **Deactivated WPC70** | Not determined |
| **Native IMF** | 33 mU/g |
| **Deactivated IMF** | < 3 mU/g |

### Example 5: Determination of alkaline phosphatase activity

The alkaline phosphatase (ALP) activity in native WPC and IMF products according to the invention (without and with denaturation) was determined in mU/L via ISO standard 11816-1 (version valid in Oct 2018). Solutions were prepared and tested according to the test protocol. The results for all four products at 1.3 wt% protein (based on total weight), which is the protein content of standard infant formulae, are given in the table below:

| | **ALP activity** |
|---|---|
| **Native WPC70** | 1.8 × 10⁴ mU/L |
| **Denatured WPC70** | < 20 mU/L |
| **Native IMF** | 2.1 × 10⁴ mU/L |
| **Denatured IMF** | < 20 mU/L |

### Example 6: Effect on gut maturation / intestinal permeability

A deactivated whey protein product with high nativity of over 90%, obtained according to the process of Example 1 was analysed with respect to its properties to improve gut maturation and intestinal permeability in piglets. *In vivo* intestinal permeability in such piglets was compared to an identical whey protein product which was denatured to a nativity level of below 40% by extensive heating as detailed below.

Piglet study: Preterm pigs (Danish Landrace x Large White x Duroc) were delivered from sows by caesarean section at approximately 90% gestation (2 litters, preterm group) and approximately 96% gestation (1 litter, near term group). Surgical preparation with an oro-gastric feeding tube and a vascular catheter for parental nutrition (PN) and passive immunization took place as previously described (Cilieborg MS, Boye M, Thymann T, et al., J Parenter Enteral Nutr. 2011;35:32-42). The pigs from each litter were stratified according to birth weight into 2 groups receiving 2 types of enteral diets (n = 7-9/group/per litter): (1) PAST-WPC group received formula based on deactivated WPC (i.e. pasteurized WPC by heating at 73°C for 30 seconds which maintains proteins in their native form; native whey protein formula groep) and (2) DENAT-WPC group received formula with WPC that was pasteurized and additionally heat-treated (i.e. heated 73°C, 30 sec + 80°C, 6 min; heated whey protein formula group), which results in extensive protein denaturation. The WPC used in this study was prepared from raw cow's milk by the process of Example 1 which did not involve heating. Each formula consisted of 80 g/L whey protein concentrate, 50 g/L Pepdite, 50 g/L Liquigen and 30 g/L Calogen. Macronutrient composition of the formulas was as follows: 3629 kJ/L Energy, 59 g/L Protein, 52 g/L Fat, 39 g/L Carbohydrate, 16 g/L Lactose. During the study period, pigs in each group received enteral nutrition as described in table below. In addition, all pigs received parenteral nutrition (PN), 4 ml/kg/h from day 1 to day 5. The piglets in both the preterm and near term litter had the same weight gain regardless the dietary group they were assigned (data not shown). PN solution was based on a commercially available product (Kabiven, Fresenius Kabi) and adjusted in nutrient composition to meet the requirement of pigs. On day 5 pigs were anaesthetized and blood was collected. Subsequently, pigs were euthanized followed by collection of urine and intestinal tissues.

Table with feeding regime:

| **Time** | **Enteral Nutrition Feeding Regime** |
|---|---|
| **Day 1** | 6 ml/kg/3h |
| **Day 2** | 8 ml/kg/3h |
| **Day 3** | 8 ml/kg/3h |
| **Day 4** | 10 ml/kg/3h |
| **Day 5** | 10 ml/kg/3h |

***In vivo* intestinal permeability test:** To test intestinal permeability, pigs received an oral bolus (15 mL/kg) containing 5% lactulose and 2% mannitol 3-5 h prior to killing. Urine samples were taken at the time of autopsy to measure the concentrations of lactulose and mannitol and the lactulose/mannitol ratio was calculated as described previously (Blood J, Ingle AR, Allison N, et al., Ann Clin Biochem. 1991;28:401-6).
**Evaluation of Gut Maturation:** To determine gut maturation status colon structure was assessed, histologic slides were assessed for Ki67 positive cells to determine the degree of proliferating cells in the crypts, presence of enteroendocrine cells and intestinal alkaline phosphatase (iALP) enzyme activity levels were determined. Paraformaldehyde-fixed colon tissue was processed for histology and stained using hematoxillin-eosin. The crypt depth was measured in µm. Histological slides were immunostained for Ki67 and proliferative cells (Ki67 positive cells) were scored in the crypts of the colon sections. Immunostaining of proliferating epithelial cells (i.e. Ki67 staining), enteroendocrine (i.e. serotonin (5HT) positive cells) and determination of brush border expression of iALP (i.e., iALP staining) immunohistochemistry was performed as described previously (Marit Navis, Tânia Martins Garcia, Ingrid B Renes et al., EMBO Reports (2018), DOI 10.15252/embr.201846221). The following antibodies were use: rabbit polyclonal anti-Ki67 (1:8000, Abcam, ab15580), and mouse monoclonal anti-5HT-H209 (1:100, ThermoFisher Scientific MA5-12111). Moreover, iALP brush border activity on tissue slides was determined with NBT/BCIP conversion as described previously (Srivillibhuthur M, Verzi et al., DOI: 10.1016/j.ydbio.2018.04.015) (Schneeberger K, Middendorp S et al., DOI: 10.1073/pnas.1516672112).

Additionally, tissue from the colon was homogenized and activity of iALP was determined by spectrophotometry with a diethanolamine assay (EC 3.1.3.1) measuring pNPP hydrolysis according to manufacturer's instruction (Phosphatase substrate, ThermoFisher Scientific) and previous described (Arnal ME, Lallès JP et al., DOI: 10.1371 /journal.pone.0118092), in presence of L-phenylalanine (Gosh NK and Fishman WH, PMID: 5911626).

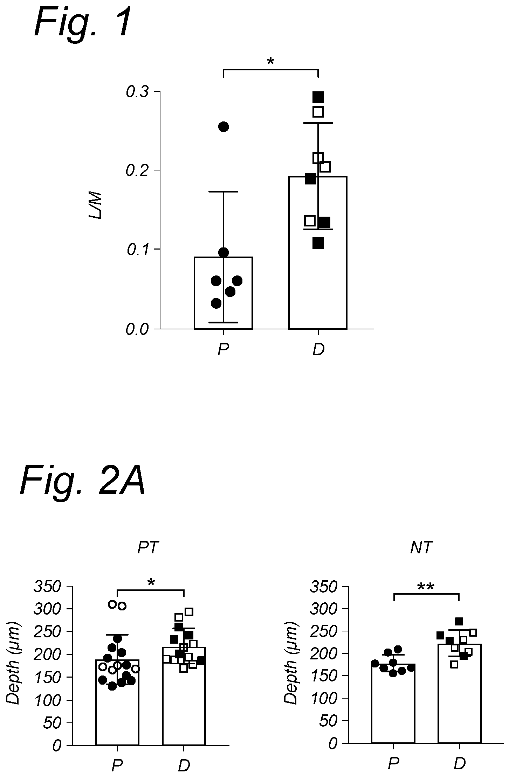

**Results:** The piglets experienced regular body weight gain which did not differ significantly between both treatment groups, nor did the weight of the colon of the piglets differ (data not shown). *In vivo* intestinal permeability based on the lactulose/mannitol (Lac/Man) ratio in urine of the piglets shows a significantly decreased (p<0.05) Lac/Man ratio in native whey protein formula treated piglets in the term group, indicative of a lower gut permeability and improved gut maturation in the native whey protein formula fed piglets (Figure 1).

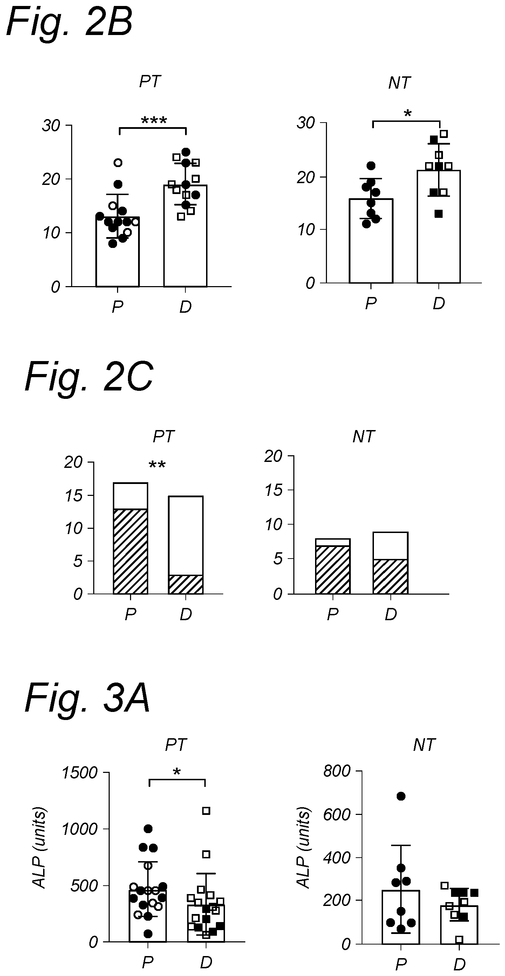

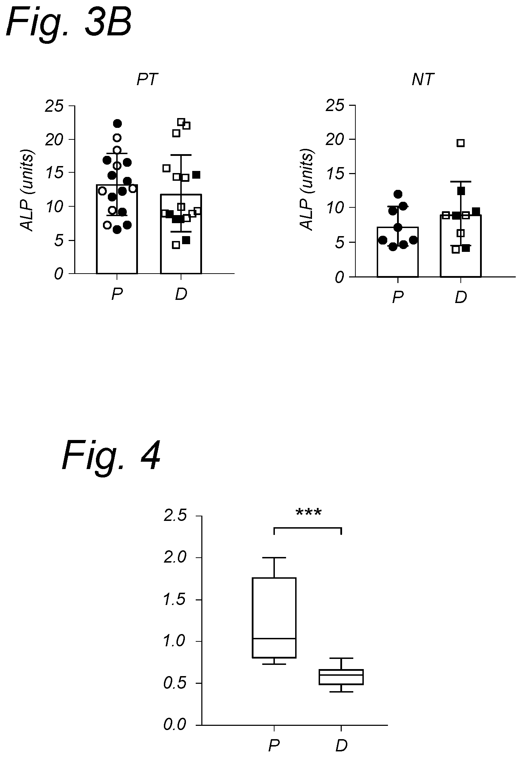

Histological analysis of the colon shows that both preterm as well as term piglets that were treated with the native whey protein formula had significantly decreased crypt depth, indicative of a more natural gut development. Formula-fed piglets are known from literature to have significantly longer villi and deeper crypts in comparison to suckled piglets (* p<0.5 in the group of preterm piglets, ** p<0.01 in the group of term piglets), a phenomenon equally observed in human infants. The decreased crypt depth in the native whey protein formula fed piglets thus at least partly reverses this formula effect on crypt depth (figure 2a). A significantly decreased number of Ki67 positive proliferating cells per crypt were observed in the native whey protein treated piglets as compared to the heated whey protein formula treated piglets both in the preterm (p<0.001) and the term ((p<0.05) groups (figure 2b). The lower level of proliferating cells per crypt is indicative of a more differentiated phenotype and improved gut maturation in the native whey protein formula fed piglets. Histological colon slides from native whey protein treated piglets had prominent apical brush border ALP activity staining (data not shown). Figure 2c shows that in the preterm group fed the native whey protein formula a significantly higher amount of animals had histological positive alkaline activity compared to the preterm animals fed the heated whey protein formula. In the piglets born term there is a trend observable that the native whey protein formula treated animals have more positive intestinal alkaline phosphatase activity than the animals treated with the heated whey protein formula. Figure 4 demonstrates that the near term piglets that received the native whey protein formula exhibited an increased enteroendocrine cell count, compared to piglets that received the heated whey protein formula, indicative of an improved gut permeability and gut maturation in subjects receiving the composition according to the invention.

Spectroscopic analysis of intestinal alkaline phosphatase (iALP) activity in colon homogenates showed a significantly increased iALP activity in the preterm piglets fed the native whey protein formula as compared to those piglets born preterm and fed the heated whey protein formula. In the near term piglets fed the native whey protein formula there is a trend towards an increased iALP activity as compared to the piglets fed the heated whey protein formula (figure 3a). No significant difference was observed in iALP activity in the samples after specific blockage of iALP between the different diet groups in either the preterm or the near term piglets (figure 3b). Increased iALP activity in the animals fed the native whey protein composition is suggestive for a more mature immune function of the gut.

### Example 7: Infant formula containing native whey protein concentrate (WPC)

An infant formula containing native whey protein according to the invention is exemplified as follows. The main nutrients of this infant formula are as follows:

| | | Units | Per 100 ml RTF | Per 100 kcal |
|---|---|---|---|---|
| Energy value | | kcal | 66 | 100 |
| Protein | | g | 1,3 | 2 |
| | -Whey | g | 0,8 | 1,2 |
| | -Casein | g | 0,5 | 0,8 |
| Carbohydrate | | g | 7,3 | 11,1 |
| | - of which sugars | g | 7,2 | 10,9 |
| | -Glucose | g | 0,2 | 0,3 |
| | -Lactose | g | 7,0 | 10,6 |
| | -Galactose | g | 0,01 | 0,02 |
| | -Polysaccharides | g | 0,01 | 0,02 |
| Fat | | g | 3,4 | 5,1 |
| - Vegetable | | g | 3,3 | 5 |
| - Animal | | g | 0,1 | 0,1 |
| - Saturated | | g | 1,5 | 2,2 |
| - Monounsaturated | | g | 1,4 | 2,1 |
| - Polyunsaturated | | g | 0,6 | 0,8 |
| Dietary fibre | | g | 0,6 | 0,9 |

The infant formula is intended for feeding of term infants aged 0 to 3 months. In terms of energy value, the infant formula contains 8 En% protein, 44 En% carbohydrate, 46 En% fat and 2 En% dietary fibre. Minerals and vitamins are included according to prevailing nutritional guidelines to produce a complete enteral infant feed. The indicated totals may not be reached due to rounding off of values. RTF = Ready-To-Feed. Whey protein is present in the infant formula with a nativity of more than 90%. The ALP activity of the RTF infant formula is either not higher than 350 mU/L and considered ALP negative or ALP positive in which case ALP activity is above 350 mU/L. Microbial safety of the ALP positive infant formula is ensured by microfiltration over a membrane capable of retaining bacteria.

### Example 8: Preterm formula containing native whey protein concentrate (WPC)

An preterm formula containing native whey protein according to the invention is exemplified as follows. The main nutrients of this preterm formula are as follows.

| | | Units | Per 100 ml RTF | Per 100 kcal |
|---|---|---|---|---|
| Energy value | | kcal | 78 | 100 |
| Protein | | g | 2,6 | 3,3 |
| | -Whey | g | 1,5 | 2,0 |
| | -Casein | g | 1,0 | 1,3 |
| Carbohydrate | | g | 8,2 | 10,4 |
| | - of which sugars | g | 6,1 | 7,7 |
| | -Glucose | g | 0,3 | 0,4 |
| | -Lactose | g | 5,5 | 6,9 |
| | -Maltose | g | 0,2 | 0,3 |
| | -Polysaccharides | g | 2,1 | 2,6 |
| Fat | | g | 3,8 | 4,8 |
| - Vegetable | | g | 3,4 | 4,2 |
| - Animal | | g | 0,3 | 0,5 |
| - Saturated | | g | 1,6 | 2,0 |
| - Monounsaturated | | g | 1,4 | 1,8 |
| - Polyunsaturated | | g | 0,8 | 1,0 |
| Dietary fibre | | g | 0,6 | 0,7 |
| Minerals | | g | 0,2 | 0,2 |

The preterm formula is intended for feeding of preterm infants, meaning infants born before the 37^{th} week of gestation. The protein amount is increased compared to infant formula intended for feeding of term infants for reasons related to catch-up growth which is intended to occur in preterm-born infants. In terms of energy value, the preterm formula contains 13 En% protein, 42 En% carbohydrate, 44 En% fat and 1 En% dietary fibre. RTF = Ready-To-Feed. Minerals and vitamins are included according to nutritional guidelines to produce a complete enteral preterm feed. The indicated totals may not be reached due to rounding off of values. Whey protein is present in the preterm formula with a nativity of more than 90%. The ALP activity of the RTF preterm formula is not higher than 350 mU/L and considered ALP negative.

## Claims

1. A native non-human whey protein having a nativity of more than 80% for use in:
(a) improving intestinal maturation; and/or
(b) reducing and/or preventing intestinal permeability,
wherein the intestine includes at least the colon.

2. The native whey protein for use according to claim 1, which is for use in reducing and/or preventing intestinal permeability.

3. The native whey protein for use according to claim 1 or 2, wherein intestinal maturation is improved in, and/or intestinal permeability is reduced and/or prevented, in a vulnerable subject, preferably an infant, more preferably a preterm infant.

4. The native whey protein for use according to any one of the preceding claims, wherein the improved intestinal maturation reduces predisposition for food intolerance and/or intestinal inflammation, such as necrotizing enterocolitis.

5. The native whey protein for use according to any one of the preceding claims, wherein the native whey protein has a nativity of more than 90%, preferably more than 95%.

6. The native whey protein for use according to any one of the preceding claims, wherein at least 70 %, more preferably 75 - 95 %, most preferably 78 - 85 % of the α-lactalbuminis native and/or at least 70 %, more preferably 80 - 100 %, most preferably 85 - 95 % of the β-lactoglobulin is native.

7. The native whey protein for use according to any one of the preceding claims, wherein the improvement of intestinal maturation, and/or the reduction and/or prevention of intestinal permeability, occurs with respect to the occurrence thereof in a subject fed the same whey protein which is made non-native, preferably by sufficient heating to reach a nativity of less than 20%.

8. The native whey protein for use according to any one of the preceding claims, wherein the intestine is the colon.

9. The native whey protein for use according to any one of the preceding claims, wherein the native whey protein is bovine whey protein obtained by a membrane-filtration based technology to retain the nativity of the whey protein.

10. The native whey protein for use according to any one of the preceding claims, wherein the native whey protein is obtainable by a process comprising:
(a) processing defatted milk into a casein stream, a whey protein stream and a lactose stream, by:
(i) subjecting the defatted milk to microfiltration over a membrane capable of retaining bacteria and permeating milk proteins or to a pasteurization step, to provide a debacterialized milk;
(ii) subjecting the permeate originating from step (i) to microfiltration over a membrane capable of retaining casein and permeating whey proteins, to provide a casein stream as retentate and a permeate comprising whey protein;
(iii) fractionating the permeate originating from step (ii) into a whey protein stream and a lactose stream.

11. The native whey protein for use according to any one of the preceding claims, wherein the native whey protein is comprised in a nutritional composition, preferably an infant formula, more preferably a preterm formula.

12. The native whey protein for use according to claim 11, wherein the nutritional composition is obtainable by a process comprising:
(a) processing defatted milk into a casein stream, a whey protein stream and a lactose stream, by:
(i) subjecting the defatted milk to microfiltration over a membrane capable of retaining bacteria and permeating milk proteins or to a pasteurization step, to provide a debacterialized milk;
(ii) subjecting the permeate originating from step (i) to microfiltration over a membrane capable of retaining casein and permeating whey proteins, to provide a casein stream as retentate and a permeate comprising whey protein;
(iii) fractionating the permeate originating from step (ii) into a whey protein stream and a lactose stream;
(b) combining at least part of the casein stream, at least part of the whey protein stream originating from step (a) and a lactose source to obtain a recombined stream;
(c) optionally pasteurization the recombined stream from step (b),
(d) using the recombined stream originating from step (b) or (c) in the manufacture of the nutritional composition.

13. The native whey protein for use according to claim 10 or 12, wherein one or more of the following applies:
- the defatted milk is defatted bovine milk.
- at least part of the lactose stream originating from step (a) is used as lactose source in step (b).
- step (iii) is performed by ultrafiltration over a membrane capable of retaining whey proteins and permeating lactose, to provide a whey protein stream as retentate and a permeate comprising lactose, preferably wherein ultrafiltration step (iii) operates with a volume concentration factor in the range of 20 - 200.
- the defatted milk is the sole protein source for the infant formula.
- the manufacturing of step (d) includes at least one of drying, concentrating, supplementing with vitamins, minerals, lipids and/or dietary fibres, packaging.
- the nutritional composition is a powder obtained by spray-drying, preferably as part of step (d).

14. The native whey protein for use according to any one of claims 11 - 13, wherein the nutritional composition is not subjected to heat treatment and/or wherein the infant formula exhibits an alkaline phosphatase activity of at least 25 mU/g.

15. The native whey protein for use according to any one of claims 11 - 13, wherein the nutritional composition is a pasteurized nutritional composition and/or wherein the nutritional composition exhibits an alkaline phosphatase activity of at most 20 mU/g, preferably at most 5 mU/g.

## Patentansprüche

1. Natives nicht-menschliches Molkenprotein mit einer Nativität von mehr als 80 % zur Anwendung in:
(a) der Verbesserung der intestinalen Reifung; und/oder
(b) der Reduzierung und/oder Vorbeugung der intestinalen Permeabilität,
wobei der Darm mindestens den Dickdarm einschließt.

2. Natives Molkenprotein zur Anwendung nach Anspruch 1, das zur Anwendung in der Reduzierung und/oder Vorbeugung der intestinalen Permeabilität dient.

3. Natives Molkenprotein zur Anwendung nach Anspruch 1 oder 2, wobei die intestinale Reifung verbessert wird und/oder die intestinale Permeabilität reduziert und/oder vorgebeugt wird bei einem vulnerablen Subjekt, vorzugsweise einem Säugling, stärker bevorzugt einem Frühgeborenen.

4. Natives Molkenprotein zur Anwendung nach einem der voranstehenden Ansprüche, wobei die verbesserte intestinale Reifung die Veranlagung für Nahrungsmittelunverträglichkeit und/oder intestinale Entzündung, wie nekrotisierende Enterokolitis, reduziert.

5. Natives Molkenprotein zur Anwendung nach einem der voranstehenden Ansprüche, wobei das native Molkenprotein eine Nativität von mehr als 90 %, vorzugsweise mehr als 95 %, aufweist.

6. Natives Molkenprotein zur Anwendung nach einem der voranstehenden Ansprüche, wobei mindestens 70 %, bevorzugter 75-95 %, am meisten bevorzugt 78-85 % des α-Lactalbumins nativ sind und/oder mindestens 70 %, bevorzugter 80-100 %, am meisten bevorzugt 85-95 % des ß-Lactoglobulins nativ sind.

7. Natives Molkenprotein zur Anwendung nach einem der voranstehenden Ansprüche, wobei die Verbesserung der intestinalen Reifung und/oder die Reduzierung und/oder Vorbeugung der intestinalen Permeabilität in Bezug auf das Auftreten derselben bei einem Subjekt auftritt, das mit demselben Molkenprotein gefüttert wird, das nicht nativ gemacht wurde, vorzugsweise durch ausreichendes Erhitzen, um eine Nativität von weniger als 20 % zu erreichen.

8. Natives Molkenprotein zur Anwendung nach einem der voranstehenden Ansprüche, wobei der Darm der Dickdarm ist.

9. Natives Molkenprotein zur Anwendung nach einem der voranstehenden Ansprüche, wobei das native Molkenprotein Rinder-Molkenprotein ist, das durch eine auf Membranfiltration basierende Technologie erhalten wurde, um die Nativität des Molkenproteins beizubehalten.

10. Natives Molkenprotein zur Anwendung nach einem der voranstehenden Ansprüche, wobei das native Molkenprotein durch ein Verfahren erhältlich ist, das Folgendes umfasst:
(a) Verarbeiten von entfetteter Milch zu einem Kaseinstrom, einem Molkenproteinstrom und einem Laktosestrom durch:
(i) Unterziehen der entfetteten Milch einer Mikrofiltration über eine Membran, die in der Lage ist, Bakterien zurückzuhalten und Milchproteine durchzulassen, oder einem Pasteurisierungsschritt, um eine entkeimte Milch bereitzustellen;
(ii) Unterziehen des aus Schritt (i) stammenden Permeats einer Mikrofiltration über eine Membran, die in der Lage ist, Kasein zurückzuhalten und Molkenproteine durchzulassen, um einen Kaseinstrom als Retentat und ein Molkenprotein umfassendes Permeat bereitzustellen;
(iii) Fraktionieren des aus Schritt (ii) stammenden Permeats in einen Molkenproteinstrom und einen Laktosestrom.

11. Natives Molkenprotein zur Anwendung nach einem der voranstehenden Ansprüche, wobei das native Molkenprotein in einer Nährstoffzusammensetzung, vorzugsweise einer Säuglingsanfangsnahrung, besonders bevorzugt einer Frühgeborenen-Nahrung, umfasst ist.

12. Natives Molkenprotein zur Anwendung nach Anspruch 11, wobei die Nährstoffzusammensetzung durch ein Verfahren erhältlich ist, das Folgendes umfasst:
(a) Verarbeiten von entfetteter Milch zu einem Kaseinstrom, einem Molkenproteinstrom und einem Laktosestrom durch:
(i) Unterziehen der entfetteten Milch einer Mikrofiltration über eine Membran, die in der Lage ist, Bakterien zurückzuhalten und Milchproteine durchzulassen, oder einem Pasteurisierungsschritt, um eine entkeimte Milch bereitzustellen;
(ii) Unterziehen des aus Schritt (i) stammenden Permeats einer Mikrofiltration über eine Membran, die in der Lage ist, Kasein zurückzuhalten und Molkenproteine durchzulassen, um einen Kaseinstrom als Retentat und ein Molkenprotein umfassendes Permeat bereitzustellen;
(iii) Fraktionieren des aus Schritt (ii) stammenden Permeats in einen Molkenproteinstrom und einen Laktosestrom.
(b) Kombinieren mindestens eines Teils des Kaseinstroms, mindestens eines Teils des Molkenproteinstroms stammenden aus Schritt (a) und einer Laktosequelle, um einen rekombinierten Strom zu erhalten;
(c) optional Pasteurisierung des rekombinierten Stroms aus Schritt (b),
(d) Verwenden des aus Schritt (b) oder (c) stammenden rekombinierten Stroms bei der Herstellung der Nährstoffzusammensetzung.

13. Natives Molkenprotein zur Anwendung nach Anspruch 10 oder 12, wobei eine oder mehrere der folgenden Bedingungen zutreffen:
- die entfettete Milch ist entfettete Kuhmilch.
- mindestens ein Teil des aus Schritt (a) stammenden Laktosestroms wird als Laktosequelle in Schritt (b) verwendet.
- Schritt (iii) wird durch Ultrafiltration über eine Membran durchgeführt, die in der Lage ist, Molkenproteine zurückzuhalten und Laktose durchzulassen, um einen Molkenproteinstrom als Retentat und ein Laktose umfassendes Permeat bereitzustellen, wobei der Ultrafiltrationsschritt (iii) vorzugsweise mit einem Volumenkonzentrationsfaktor im Bereich von 20-200 arbeitet.
- die entfettete Milch ist die einzige Proteinquelle für die Säuglingsanfangsnahrung.
- die Herstellung aus Schritt (d) schließt mindestens eines von Trocknen, Konzentrieren, Ergänzen mit Vitaminen, Mineralien, Lipiden und/oder Ballaststoffen, Verpacken ein.
- die Nährstoffzusammensetzung ist ein Pulver, das durch Sprühtrocknung, vorzugsweise als Teil von Schritt (d), erhalten wird.

14. Natives Molkenprotein zur Anwendung nach einem der Ansprüche 11-13, wobei die Nährstoffzusammensetzung keiner Wärmebehandlung unterzogen wird und/oder wobei die Säuglingsanfangsnahrung eine Alkaliphosphataseaktivität von mindestens 25 mU/g aufweist.

15. Natives Molkenprotein zur Anwendung nach einem der Ansprüche 11-13, wobei die Nährstoffzusammensetzung eine pasteurisierte Nährstoffzusammensetzung ist und/oder wobei die Nährstoffzusammensetzung eine alkalische Phosphataseaktivität von höchstens 20 mU/g, vorzugsweise höchstens 5 mU/g, aufweist.

## Revendications

1. Protéine de lactosérum non humaine, native, ayant une nativité de plus de 80 % pour une utilisation pour :
(a) améliorer la maturation intestinale ; et/ou
(b) réduire et/ou prévenir la perméabilité intestinale,
l'intestin comprenant au moins le côlon.

2. Protéine de lactosérum native pour une utilisation selon la revendication 1, qui est destinée à être utilisée pour réduire et/ou prévenir la perméabilité intestinale.

3. Protéine de lactosérum native pour une utilisation selon l'une des revendications 1 ou 2, dans laquelle la maturation intestinale est améliorée et/ou la perméabilité intestinale est réduite et/ou prévenue chez un sujet vulnérable, de préférence un nourrisson, de façon davantage préférée un nourrisson prématuré.

4. Protéine de lactosérum native pour une utilisation selon l'une quelconque des revendications précédentes, dans laquelle la maturation intestinale améliorée réduit la prédisposition à l'intolérance alimentaire et/ou à l'inflammation intestinale, telle que l'entérocolite nécrosante.

5. Protéine de lactosérum native pour une utilisation selon l'une quelconque des revendications précédentes, dans laquelle la protéine de lactosérum native a une nativité de plus de 90 %, de préférence de plus de plus de 95 %.

6. Protéine de lactosérum native pour une utilisation selon l'une quelconque des revendications précédentes, dans laquelle au moins 70 %, de façon davantage préférée de 75 à 95 %, de la façon que l'on préfère le plus de 78 à 85 %, de l'α-lactalbumine est native et/ou au moins 70 %, de façon davantage préférée de 80 à 100 %, de la façon que l'on préfère le plus de 85 à 95 %, de la β-lactoglobuline est native.

7. Protéine de lactosérum native pour une utilisation selon l'une quelconque des revendications précédentes, dans laquelle l'amélioration de la maturation intestinale, et/ou la réduction et/ou la prévention de la perméabilité intestinale, a lieu par rapport à leur apparition chez un sujet nourri avec la même protéine de lactosérum qui est rendue non native, de préférence par un chauffage suffisant pour atteindre une nativité inférieure à 20 **%.**

8. Protéine de lactosérum native pour une utilisation selon l'une quelconque des revendications précédentes, dans laquelle l'intestin est le côlon.

9. Protéine de lactosérum native pour une utilisation selon l'une quelconque des revendications précédentes, dans laquelle la protéine de lactosérum native est une protéine de lactosérum bovin obtenue par une technologie basée sur la filtration sur membrane pour conserver la nativité de la protéine de lactosérum.

10. Protéine de lactosérum native pour une utilisation selon l'une quelconque des revendications précédentes, dans laquelle la protéine de lactosérum native est susceptible d'être obtenue par un procédé comprenant :
a) transformer du lait dégraissé en un courant de caséine, un courant de protéine de lactosérum et un courant de lactose, en :
(i) soumettant le lait dégraissé à une microfiltration sur une membrane capable de retenir les bactéries et de réaliser la perméation des protéines de lait ou à une étape de pasteurisation, pour fournir un lait débactérisé ;
(ii) soumettant le perméat provenant de l'étape (i) à une microfiltration sur une membrane capable de retenir la caséine et de réaliser la perméation des protéines de lactosérum, pour fournir un courant de caséine comme rétentat et un perméat comprenant de la protéine de lactosérum ;
(iii) fractionnant le perméat provenant de l'étape (ii) en un courant de protéine de lactosérum et un courant de lactose.

11. Protéine de lactosérum native pour une utilisation selon l'une quelconque des revendications précédentes, dans laquelle la protéine de lactosérum native est comprise dans une composition nutritionnelle, de préférence une préparation pour nourrissons, de façon davantage préférée une préparation pour prématurés.

12. Protéine de lactosérum native pour une utilisation selon la revendication 11, dans laquelle la composition nutritionnelle est susceptible **d'être** obtenue par un procédé comprenant :
a) transformer du lait dégraissé en un courant de caséine, un courant de protéine de lactosérum et un courant de lactose, en :
(i) soumettant le lait dégraissé à une microfiltration sur une membrane capable de retenir les bactéries et de réaliser la perméation des protéines de lait ou à une étape de pasteurisation, pour fournir un lait débactérisé ;
(ii) soumettant le perméat provenant de l'étape (i) à une microfiltration sur une membrane capable de retenir la caséine et de réaliser la perméation des protéines de lactosérum, pour fournir un courant de caséine comme rétentat et un perméat comprenant de la protéine de lactosérum ;
(iii) fractionnant le perméat provenant de l'étape (ii) en un courant de protéine de lactosérum et un courant de lactose ;
b) combiner au moins une partie du courant de caséine, au moins une partie du courant de protéine de lactosérum provenant de **l'étape** (a) et une source de lactose pour obtenir un courant recombiné ;
c) facultativement pasteuriser le courant recombiné provenant de l'étape (b) ;
d) utiliser le courant recombiné provenant de l'étape (b) ou (c) dans la fabrication de la composition nutritionnelle.

13. Protéine de lactosérum native pour une utilisation selon l'une des revendications 10 ou 12, dans laquelle au moins l'un de ce qui suit s'applique :
- le lait dégraissé est du lait bovin dégraissé ;
- au moins une partie du courant de lactose provenant de l'étape (a) est utilisée comme source de lactose dans l'étape (b) ;
- l'étape (iii) est réalisée par ultrafiltration sur une membrane capable de retenir les protéines de lactosérum et de réaliser une perméation du lactose, pour fournir un courant de protéine de lactosérum comme rétentat et un perméat comprenant du lactose, de préférence où l'étape d'ultrafiltration (iii) fonctionne avec un facteur de concentration volumique dans la plage de 20 à 200 ;
- le lait dégraissé est la seule source de protéines pour la préparation pour nourrissons ;
- la fabrication de l'étape (d) comprend au moins l'un parmi le séchage, la concentration, la supplémentation en vitamines, minéraux, lipides et/ou fibres alimentaires, l'emballage ;
- la composition nutritionnelle est une poudre obtenue par séchage par pulvérisation, de préférence dans le cadre de l'étape (d).

14. Protéine de lactosérum native pour une utilisation selon l'une quelconque des revendications 11 à 13, dans laquelle la composition nutritionnelle **n'est** pas soumise à un traitement thermique et/ou dans laquelle la préparation pour nourrissons présente une activité phosphatase alcaline d'au moins 25 mU/g.

15. Protéine de lactosérum native pour une utilisation selon l'une quelconque des revendications 11 à 13, dans laquelle la composition nutritionnelle est une composition nutritionnelle pasteurisée et/ou dans laquelle la composition nutritionnelle présente une activité phosphatase alcaline d'au plus 20 mU/g, de préférence d'au plus 5 mU/g.
